# EUROPÄISCHE PATENTSCHRIFT

(11) **EP 0 330 843 B1**
(45) Veröffentlichungstag und Bekanntmachung des Hinweises auf die Patenterteilung: **22.12.1993**
(21) Anmeldenummer: 89101528.1
(22) Anmeldetag: 30.01.1989
(51) Int. Cl.: A61B 17/22

(54) **Verfahren und Vorrichtung zum Entfernen von Ablagerungen in Gefässen und Organen von Lebewesen**
Method and apparatus for removing deposits from blood vessels and organs of living beings
Procédé et dispositif pour enlever des dépôts dans les vaisseuax sanguins et les organes d'êtres vivants

(30) Priorität: 04.03.1988 DE 8802950 U; 28.07.1988 DE 8809604 U; 08.12.1988 DE 8815251 U
(43) Veröffentlichungstag der Anmeldung: 06.09.1989
(73) Patentinhaber: ANGIOMED Aktiengesellschaft, 76229 Karlsruhe (DE)
(72) Erfinder: Schnepp-Pesch, Wolfram, 7505 Ettlingen (DE); Lindenberg, Josef, 7500 Karlsruhe (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- US-A- 3 732 858
- US-A- 3 976 077
- US-A- 4 320 761
- US-A- 4 706 671

## Beschreibung

Die Erfindung betrifft eine Vorrichtung zum Entfernen von Ablagerungen, wie Plaques in Gefäßen und Steine in Organen von Lebewesen, mit einem hohlen Führungsteil, einem Antrieb und einer von diesem angetriebenen, ins Führungsteil ragenden Welle, wobei das distale Ende des Wendelansatzes abgestumpft ausgebildet ist und der äußere Bereich der einzelnen Windungen ebenfalls abgestumpft ist.

Bei operativer Gallensteinentfernung ist ein sehr großer Schnitt zu setzen, der oft schlecht heilt und beträchtliche Narbenplatten bildet. Aufgrund der hohen Rezidivquote der Gallensteinbildung wird zur Vermeidung wiederholten Entfernens derartiger Steine noch in vielen Fällen eine operative Entfernung der gesamten Gallenblase erforderlich. Abgesehen davon, daß eine solche Operation außerordentlich belastend ist und eine erhebliche Aufenthaltsdauer im Krankenhaus verlangt, ist sie risikoreich. Sie muß unter Vollnarkose durchgeführt werden, die immer ein erhebliches Risiko bildet. Insbesondere können weiche Steine, wie Cholesterin - und Pigmentsteine auch nicht mittels Ultraschallzertrümmerung zerkleinert und entfernt werden. Es wurde weiterhin versucht, Gallensteine mittels Laserstrahlen zu zerstören, was aber ebenfalls nicht bei allen Steinarten optimal ist.

Es wurde versucht, Ablagerungen in Blutgefäßen mittels eines in einem mit einem seitlichen Fenster versehenen Katheter um laufenden Messers zu entfernen. Befriedigende Ergebnisse konnten hiermit nicht erreicht werden.

Die US-A-4 706 671 zeigt eine Vorrichtung zum Entfernen von Ablagerungen, wie Plaques in Gefäßen mit einem Katheter aus einem durch eine Nadel geführten weichen Katheterkörper, der bis zu einem gewissen Grad versteift ist.. Es ist weiterhin eine Hohlwendel aus Extrusionsmaterial vorgesehen, die bei Einführung gestreckt im Katheterkörper ausgebildet und nach Einführen aus diesem herausfahrbar ist und ihre Wendelform annehmen kann. Während der zunächst auch in dieser befindliche Führungsdraht entfernt wird, kann die Hohlwendel durch ein Fluid versteift werden, um dann, wenn sie zurückgezogen wird, Ablagerungsmaterial zum Katheterkörper und der Hohlwendel zu bewegen, wo dieses dann manuell oder durch Absaugen entfernt werden kann. Die Vorrichtung mag zum Heranbringen losen Ablagerungsmaterials zur Nadel zwecks Absaugen geeignet sein, mit dieser weichen und streckbaren Hohlwendel dürfte aber ein Lösen von Plaques von den Wandungen des Gefäßes selbst nicht möglich sein.

In üblicher Weise werden Thromben mittels einer Atherektomie-Schlinge gefaßt, soweit möglich zerkleinert und durch einen Führungskatheter herausgezogen, wobei dies durch Absaugen unterstützt werden kann. Dieses Verfahren ist aufwendig und erfordert vom Operateur ein erhebliches Feingefühl. Es kann leicht geschehen, daß ein abgetrennten Thrombenteil aus der Schlinge herausrutscht, weitergetragen wird und engere Gefäße verstopft.

Der Erfindung liegt daher die Aufgabe zugrunde, eine Vorrichtung und ein Verfahren derartiger Ablagerungen zu schaffen, mittels derer die Notwendigkeit von Gallenblasen-Operationen auch bei weichen Steinen vermieden, zumindestens weiter wesentlich reduziert wird und mit dem sowohl Thromben als auch schon durch Ablagerungen entstehende Blutgefäßverengungen beseitigt werden können.

Erfindungsgemäß wird die genannte Aufgabe durch eine Vorrichtung zum Entfernen von Ablagerungen, wie Plaques in Gefäßen und Steinen in Organen von Lebewesen, mit einem hohlen Führungsteil, einem Motor und einer von diesem angetriebenen, ins Führungsteil ragenden Welle, wobei am distalen Ende der Antriebswelle ein Wendelansatz angeordnet ist, der das distale Ende des Führungsteils überragt und deren Querschnittabmessungen höchstens dem Innendurchmesser des Führungsteils entsprechen, gelöst, wobei das distale Ende des Wendelansatzes abgestumpft ausgebildet ist und der äußere Bereich der einzelnen Windungen ebenfalls abgestumpft ist. Die Bezeichnungen "distal" und "proximal" sind in der Medizintechnik eindeutig, sie beziehen sich bei Geräten und Vorrichtungen auf den Arzt bzw. Operateur (im Gegensatz zu Körperteilen, wo der Bezug das Herz des Patienten ist).

Die Erfindung ermöglicht in einfacher Weise nicht nur die Entfernung von Ablagerungen in Gefäßen, sondern nimmt entfernte Ablagerungsteile durch die Wendelausbildung auf und führt diese sicher zum und in das distale Ende des hohlen Führungsteils. Ein wesentlicher Vorteil der Erfindung liegt darin, daß die Zerstörung und Entfernung der Ablagerungen unter Lokalanästhesie erfolgen kann.

Die Entfernung der Ablagerungsteile kann insbesondere durch Absaugung unterstützt werden. Weiterhin ermöglicht die Erfindung die Zerstörung von Gallensteinen nach Punktion der Galle, indem der Wendelansatz gegen einen derartigen Stein gedrückt wird und ihn unter der vom Motor über die Welle ausgeübten Drehung zerstört. Auch hier kann eine Absaugung vorgesehen sein.

Der Wendelansatz ist vorzugsweise, wie auch die Welle, starr ausgebildet. Die Welle ist dennoch notwendigenfalls in ihrem im Führungsteil befindlichen Bereich biegbar. Auch in diesem Falle rotiert der Wendelansatz aufgrund der Führung der Welle durch das Führungsteil um seine Achse.

In bevorzugter Ausgestaltung ist vorgesehen, daß die einzelnen Windungen des Wendelansatzes mit endlichem Abstand zueinander angeordnet sind. Hierdurch können Ablagerungsteil zwischen den einzelnen Windungen der Wendel aufgenommen und durch diese zum distalen Ende des Führungsteils zuverlässig gefördert werden. Dies kann weiterhin dadurch unterstützt werden, daß die Windungen des Wendelansatzes, insbesondere ein durch deren Innenwandbereiche begrenzter Rotationsellipsoid einen Freiraum innerhalb des Wendelansatzes umgeben, wobei dann insbesondere Ablagerungsteile innerhalb der Wendel zum Führungsteil hingefördert werden können.

Eine weitere bevorzugte Ausbildung sieht vor, daß ein Wendelansatz als auf einem axialen Kern ausgebildetes Schraubengewinde ausgebildet ist, wobei insbesondere das distale (vordere) Ende der Welle eine blattförmige Verbreiterung aufweist und weiterhin die blattförmige Verbreiterung in einer der Schraubenform entsprechenden Weise um die Symmetrieachse der Welle in sich gebogen ist.

Die Ausgestaltung des Wendelansatzes kann unterschiedlich sein. So sieht eine bevorzugte Ausgestaltung vor, daß der Querschnitt des Wendelansatzes über dessen Höhe konstant ist. Eine derartige Ausgestaltung wird insbesondere bei der Entfernung von Ablagerrungen in Gefäßen eingesetzt. Eine andere vorteilhafte Ausgestaltung sieht vor, daß der Wendelansatz konisch, insbesondere sich zu seinem distalen Ende hin verjüngend ausgebildet ist. Eine derartige Ausgestaltung ist insbesondere in Weiterbildung mit einer Verjüngerung zum distalen Ende hin vorteilhaft, da der Wendelansatz sich in den Stein bohren und diesen aufsprengen kann. Bei einer Erweiterung des Wendelansatzes zum distalen Ende, die auch grundsätzlich möglich ist, könnten Steinteile in dem Wendelbereich gefangen und verklemmt und dann mit dem gesamten Wendelansatz herausgezogen werden. Gemäß einer anderen Ausbildung ist vorgesehen, daß das distale Ende des Wendelansatzes aus der Achsrichtung der Antriebswelle leicht herausgebogen ist. Auch diese Ausgestaltung ist insbesondere bei der Zerstörung von Steinen vorteilhaft, da hiermit periodisch mit dem abgebogenen distalen Ende des Wendelansatzes gegen den Stein geschlagen und dieser dadurch zerstört werden kann. Die Zertrümmerung von Gallensteinen erfolgt selbstverständlich unter Beobachtung, vorzugsweise mittels röntgenologischer Durchleuchtung oder Ultraschall.

Um Verletzungen, insbesondere bei empfindlichen Blutgefäßen, zuverlässig auszuschließen, ist der distale Bereich des Wendelansatzes abgestumpft ausgebildet, wobei insbesondere das distale Ende des Wendelansatzes umgebogen und das distale Ende des Wendelrads in Form einer Acht ausgebildet sein könnte.

Während Welle und Wendelansatz grundsätzlich separat ausgestaltet sein können und dann mit aneinander angepaßten Befestigungseinrichtungen versehen sind, so daß bei einer Welle unterschiedliche Ansätze angesetzt werden können, sieht eine bevorzugte Ausgestaltung vor, daß Welle und Wendelansatz einstückig ausgebildet sind. In Weiterbildung ist bevorzugt vorgesehen, daß das proximale Ende des Führungsteils gegen die Welle abgedichtet ist. Eine solche Ausgestaltung ist grundsätzlich vorteilhaft, wird aber insbesondere gewählt, wenn vorgesehen ist, daß das Führungsteil proximal einen von einem die Welle aufnehmenden Lumen abzweigenden Abzweig aufweist, der mit einer Saugeinrichtung verbindbar ist. Eine Weiterbildung sieht vor, daß das Führungsteil zweilumig ausgebildet ist und neben einem Hauptlumen mit nahezu den gesamten Innenquerschnittsbereich des Führungsteils einnehmenden Querschnitt ein Nebenlumen mit einem wesentlich geringeren Querschnitt aufweist, an dessen proximalem Ende, ggfls. über einen Abzweig eine Spritze ansetzbar ist. Die Ablagerungen können derart durch Steptokinase angelöst werden. Auch in diesem Fall braucht der Außendurchmesser des Führungsteils nicht über 5 mm zu liegen, kann vorzugsweise 3 bis 4 mm betragen.

Die Umdrehung des Antriebsmotors liegt vorzugsweise in der Regel von einigen zigtausend Umdrehungen, insbesondere bei ca. 20.000 Umdrehungen.

Eine weitere bevorzugte Ausgestaltung sieht vor, daß der Wendelansatz durch das Führungsteil bis in den Bereich einer Ablagerung einbringbar ist und durch Rotation des Wendelansatzes die Ablagerung abtragbar ist.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnung im einzelnen erläutert sind. Dabei zeigt bzw. zeigen:
- Figur 1: Eine Seitenansicht, teilweise geschnitten mit einer bevorzugten Ausgestaltung der erfindungsgemäßen Vorrichtung;
- Figur 2: in Vergrößerung die Wendelausgestaltung der Vorrichtung nach der Figur 1 mit distal vorgeordneter Kugel; und
- Figuren 3-7: verschiedene weitere Ausbildungen von Wendelausbildungen.

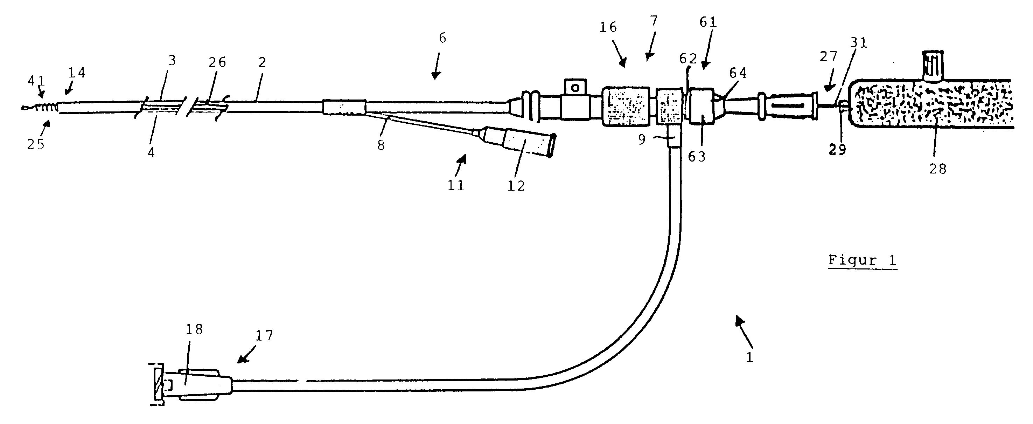

Die in der Figur 1 dargestellte Vorrichtung zum Entfernen von Ablagerungen aus Gefäßen oder Organen, wie Blutgefäßen oder der Gallenblase weist als Führungsteil 2 einen Kunststoffkatheter auf, der im dargestellten Ausführungsbeispiel zweilumig mit einem nahezu den gesamten Hohlraum des Katheters 2 ausfüllenden Hauptlumen 3 und einem weiteren Lumen 4 mit einem wesentlich geringeren Querschnitt.

Im proximalen Bereich 6 weist das Führungsteil 2 seitlich des koaxialen Endes 7 Abzweige 8,9 auf. Der Abzweig 8 mündet in das Lumen 4 und weist an seinem proximalen Ende 11 einen Adapter 12 zum Ansatz einer Spritze oder dergleichen auf, mit der Streptokinase od.dgl. durch das Lumen 4 bis zum distalen Ende 14 des Führungsteils 2 und aus diesem heraus zum Anlösen der Ablagerungen gedrückt werden kann.

Der andere Abzweig 9 ist mit dem Hauptlumen 3 des Führungsteils 2 verbunden. An seinem im Verbindungsbereich 16 mit dem Führungsteil 2 abgewandten Ende 17 über einen Adapter 18 ist eine Absaugeinrichtung zum Absaugen von in noch zu beschreibender Weise abgetrennten oder zerkleinerten Ablagerungsteilen aus dem Gefäß oder dem Organ anbringbar. Die Absaugeinrichtung kann in einfachster Ausgestaltung eine Medizinalspritze sein, die vom Arzt oder einem Helfer aufgezogen wird. In äußerst bevorzugter Ausgestaltung kann die Absaugeinrichtung eine motorisch, insbesondere elektrisch angetriebene Pumpe sein. Die Absaugrate ist dabei veränderbar, wie einstellbar, steuerbar oder regelbar. Insbesondere kann die aus dem Hauptlumen 2 abgesaugte Menge verändert werden.

Die Stirnseite 21 des Führungsteils 2 ist mit einer Dichtung abgedichtet, wie in der aus der WO-A-86/06951 bekannten Weise. Das Führungsteil 2 kann hierzu am proximalen Ende 7 mit einem Luer-Adapter 23 od.dgl. versehen sein.

In bevorzugter Ausgestaltung ist die Dichtung derart ausgebildet, daß in einem proximal des Abzweigs 9 eines Ventilteils 61, wie eines haemostatischen Ventils ausgebildeten Hülsenteil 62, ein kurzes, elastisches und flexibles Schlauchteil angeordnet ist, dessen Durchmesser im unbelasteten Zustand derart ist, daß ein am distalen Ende 25 einer im Führungsteil 2 liegenden Antriebswelle 26 in Form eines Drahtes ausgebildeter Wendelansatz 41 frei durch das Schlauchstück hindurchgesteckt werden kann. Der Innenquerschnitt des Schlauchstücks ist also im unbelasteten Zustand mindestens so groß wie der Querschnitt des Wendelansatzes 41.

Der Hülsenansatz 62 ist mit einem Außengewinde versehen. Auf ihm sitzt eine mit einem Innengewinde versehene Kappe 63 auf. Die Kappe 63 drückt mit ihrer Stirnseite 64, wenn sie zum Abzweig 9 hingeschraubt wird, das Schlauchstück zusammen, wodurch dieses sich an der Welle 26 anlegt und eine sichere zuverlässige Abdichtung bildet. Die Welle 26 kann unter Reibung axial hin- und hergeschoben werden.

Das Führungsteil 2 wird also in seinem Hauptlumen 3 durch die Welle 26 durchragt, die mit ihrem proximalen Ende 27 durch die Dichtung 22 hindurchgeführt ist, wobei die Abdichtung zwischen Dichtung 22 und Welle 26 aufrechterhalten bleibt. Das proximale Ende 27 ist mit einem Motor 28 verbunden.

Zur Verbindung können entsprechende geeignete Bohreraufnahmen 29 vorgesehen sein, wobei der Aufnahmebereich 31 der Welle 26 zugsweise keinen größeren, zumindest keinen wesentlich größeren Querschnitt als die Welle über ihre übrige Länge hin aufweist, so daß die Welle 26 mit ihrem proximalen Ende 27 beispielsweise zunächst von der Seite der Dichtung 22, die nach Anbringung derselben am Führungsteil 2 der Stirnseite 21 desselben zugewandt ist, auch durch eine Dichtung nach der WO-A-86/06951 hindurchgesteckt werden kann. In diesem Falle wird dann anschließend die Welle 26 mit auf ihr aufsitzender Dichtung 22 in das Führungsteil 2 eingeführt (vom proximalen Ende 7 her). Die Dichtung tritt dabei in den Adapter 23 ein und wird in diesem in der dargestellten Weise durch ein mit dem Adapter 23 verbindbares Spannteil 32 festgespannt. Hierdurch ist eine zuverlässige Dichtung erreichbar.

Unabhängig von Art und Ausgestaltung der Dichtung und Hindurchbringen der Wendel durch diese, können anschließend Welle 26 und Spannansatz 29 des Motors 28 miteinander verbunden werden. Der Motor kann, wie dies dargestellt ist, ein freihändig haltbarer Handmotor sein. Er könnte aber auch mit dem Adapter 23 oder im Spannteil 32 verbindbar sein, wodurch er durch diese drehfest gehalten würde, wobei dann auch die Abdichtung im Verbindungsbereich vorgesehen sein könnte.

Bei der vorstehend beschriebenen Ausgestaltung des proximalen Endes 27 der Welle 26 und der hiermit verbundenen beschriebenen Einsatzweise ist der Wendelansatz 41 prinzipiell einstückig mit der Welle 26 ausgebildet. Alternativ können Welle 26 und Wendelansatz 41 auch über Adapterteile miteinander verbunden sein, wobei diese ebenfalls in an sich bekannter Weise ausgebildet sind. Der Adapterbereich der Welle 26 kann einen die Stärke der Welle 26 selbst nicht übertreffenden Querschnitt haben, könnte beispielsweise durch ein Außengewinde am distalen Ende 25 der Welle 26 ausgebildet sein, wobei das Adapterteil der Wendel 41 eine Hülse mit einem angepaßten Innengewinde ist. Die Gewinderichtung entsprechend ausgebildeter Adapterteile ist dabei derart vorzusehen, daß die Gewindeverbindung sich bei der üblichen Drehrichtung des Motors 28 nicht löst, sondern vielmehr festzieht.

Wenn der Wendelansatz 41 von seinem Übergangs- oder Verbindungsbereich mit der Welle 26, also seinem proximalen Ende her eine Rechtsschraube bildet, so muß die Welle 26 bei Betrachtung von ihrem proximalen Ende 27 her ebenfalls nach rechts, also im Uhrzeigersinn, umlaufen. In diesem Falle ist auch das Außengewinde am distalen Ende 25 der Welle 26 ein gleiches Rechtsgewinde. Die Gewinderichtung entspricht also jeweils der Umlaufrichtung des Wendelansatzes 41 bei gleicher Betrachtungsrichtung.

Der Wendelansatz 41 besteht aus relativ sehr steifem, wendelförmig geformtem Material unter Freilassung eines durch die einzelnen Windungen 47 bzw. deren inneren Rotationsellipsoid umschlossenen Freiraums 48 im Inneren des Wendelansatzes 41. Das distale Ende 49 des Wendelansatzes 41 ist abgestumpft ausgebildet, beispielsweise, indem es zu einer Acht 51 ("8") gebogen ist, wie dies in den Zeichnungen dargestellt ist. Der äußere Bereich der einzelnen Windungen 47 ist ebenfalls abgestumpft, vorzugsweise abgerundet. Es kann grundsätzlich steifes Runddrahtmaterial für den Wendelansatz 41 verwendet werden.

Statt einer zylindrischen Ausgestaltung der Wendel 41 können auch sich konisch zum distalen Ende 49 hin verjüngende Wendelansatz-Ausbildungen vorgesehen sein. Auch könnte der distale Bereich 49 des Wendelansatzes 41 gegenüber dessen Verbindungsbereich 42 mit der Welle 26 und damit der Achse der Welle 26 seitlich abgebogen sein.

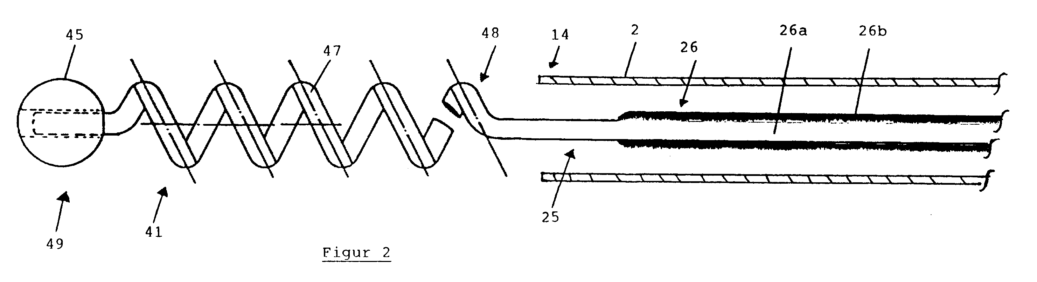

Die Figur 2 zeigt eine bevorzugte Ausgestaltung mit einem Wendelansatz 41 mit über seine Länge oder Höhe hin konstantem Querschnitt. Stattdessen kann sich die Wendel auch zum distalen Ende hin verjüngern. Der Wendelansatz 41 geht mit seinem proximalen Ende einstückig in die Welle 26 über. Am freien distalen Ende 49 ist der Wendelansatz 41 mit einer Kugel 45 versehen. Die Kugel kann mit dem Wendelansatz 41 verschweißt sein. Sie könnte mit ihm auch durch eine Schraubverbindung verbunden sein. Der Wendelansatz 41 selbst ist hier einstückig bis zum Antriebsmotor 28 reichenden Kern 26a der Welle 26 ausgebildet. Zur Versteifung ist die Welle 26 proximal zur Wendel 41 zusätzlich mit einer den Wellenkern 26a umgebenden Hülse 26b versehen, die ebenfalls bis zum Motor 28 reicht. Kern 26a und Hülse 26b sind vorzugsweise miteinander verschweißt.

Die Kugel 45 weist einen Durchmesser auf, der, ebenso wie der Durchmesser der Wendel 41, geringfügig unterhalb dem des Innenlumens des Führungsteils 2 liegt. Durch diese Kugelausgestaltung, die aufgrund ihrer Trägheit und der Flexibilität der Welle 64 sich um einen, außerhalb ihres Mittelpunkts liegenden, Drehpunkt bewegt, können Schlacken oder Ablagerungen zerschlagen werden. Sie werden dann durch den am Führungsteil 2 angelegten Unterdruck des Führungsteils 2 eingesogen und dort durch die Schraubgewindewendel 63 zum proximalen Ende hingefördert.

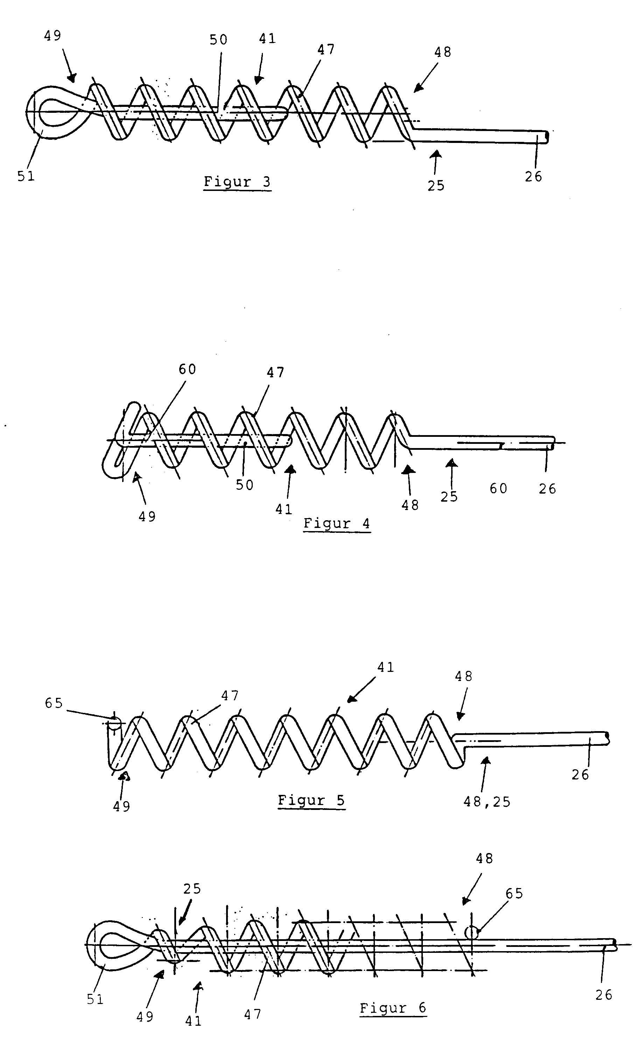

Bei der Ausgestaltung der Figur 3 ist die Kugel dadurch ersetzt, daß das freie distale Ende 49 des Wendelansatzes 41 in einer (Teil-)Acht 51 herumgebogen und mit einem geraden Endstück 50 ins Innere der Wendel zurückgeführt ist.

Die Figur 4 zeigt eine Wendel 41 mit konstantem Querschnitt über die Länge, bei der das distale Ende 49 in einer Ebene geführt ist, deren Normale einen Winkel < 90 Grad, vorzugsweise < 45 Grad, im dargestellten Ausführungsbeispiel etwa 20 bis 25 Grad, mit der Achse 60 bzw. der Antriebswelle 26 einschließt. Das distale Ende 49 erstreckt sich bei dieser Ausgestaltung radial etwas über die Stärke der Wendel 41 selbst hinaus. Ein derart ausgestaltetes distales Ende 49 könnte auch durch eine in der genannten Ebene liegende Acht gebildet sein. Stattdessen kann die durch den Wendeldraht gebildete Endabschlußfläche auch senkrecht zur Achse 60 stehen, ihre Normale also mit der Achse 60 zusammenfallen.

Die Ausgestaltung der Figur 5 weist über die Länge hin konstanten Querschnitt auf, während sich die Wendel auch wieder zum distalen Ende hin verjüngen könnten. Der Wendeldraht endet hier distal ohne eine besondere Ausgestaltung des distalen Bereichs 49. Allerdings sollte die Stirnseite 65 des freien Endes des Drahtes abgestumpft sein, indem Schneidgrate abgeschliffen sind od.dgl.

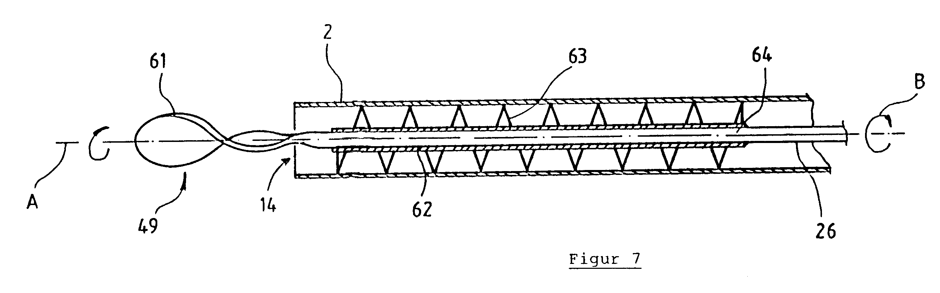

Die Ausgestaltung der Fig.6 verjüngt sich zum distalen Ende hin, während auch ein konstanter Querschnitt über die Höhe der Wendel hin gegeben sein könnte. Das distale Ende der Wendel 41 ist jeweils zu einer (Teil-)Acht 51 geformt. Der Unterschied dieser Ausgestaltung zu der Ausgestaltung der Figuren 1 bis 3 liegt im wesentlichen darin, daß die Welle 26 zunächst mit ihrem distalen Ende 25 gestreckt ausgeführt ist, sich an das distale Ende 25 die (Teil-)Acht 51 anschließt und die Wendel 41 dann um das distale Ende 25 der Welle 26 herumgewendelt ist und ihr proximales Ende 48 das freie Ende, das in ähnlicher Weise wie bei den Figuren 6 und 7, aber proximal endet.

Eine weitere Ausbildung von Wendelausgestaltungen ist der Figur 7 zu entnehmen. Diese Ausgestaltung ist ebenfalls im Zusammenhang mit den sonstigen Merkmalen der Figur 1 zu sehen. Bei dieser Ausgestaltung ist die Antriebswelle 26 einstückig ausgebildet; sie setzt sich bis zu ihrem proximalen Ende 27, mittels dessen sie mit dem Motor verbunden ist (hierzu wirde auf die Ausführungen zu Fig.1 verwiesen, einstückig fort. Sie weist an ihrem distalen Ende eine blattartige Verbreiterung 61 auf, die in sich um die Achse A der Welle 26 verdreht oder verdrillt ist, so daß sich eine teilschraubenförmige Ausbildung der Verbreiterung 61 ergibt. Im Anschluß an die Verbreiterung 61 sitzt in proximaler Richtung eine Hülse 62 auf der Welle 26, deren Innendurchmesser im wesentlichen dem Außendurchmesser der Welle 26 entspricht und die hier auf ihrer Außenseite mit einer Wendelausbildung in Form eines Schraubengewindes 63 versehen ist. Die verdrillte Verbreiterung 61 und die Schraubenausbildung 63 steigen in gleicher Richtung an und zwar derart, daß durch die Verbreiterung 61 verkleinertes und zum Einlaß des distalen Endes 25 des Führungsteils 2 gefördertes Ablagerungsmaterial bei der Drehrichtung durch die Schraubenausbildung 63 innerhalb des Führungsteils 2 in proximale Richtung gefördert wird. Die Hülse 62 ist mit der Welle 26 fest verbunden, beispielsweise durch Verschweißungsstellen 64.

In einer Variation der vorstehend beschriebenen Ausgestaltung ist die blattartige Verbreiterung 61 durch eine mit der Welle 64 verbundenen Kugel 66 ersetzt.

Es sind auch weitere Ausbildungen dieser Ausgestaltung denkbar, bei denen beispielsweise Verbreiterungen 61 und Schraubenausbildungen 63 einstückig, ggfls. auch einstückig mit der Welle 26 ausgebildet sind.

In bevorzugter Weise weisen die Wendeln 41 etwa 5 bis 10 Einzelwindungen 47 auf. Der Durchmesser der Wendeln 41, 61 bestimmt sich nach dem Einsatzgebiet und liegt in der Größenordnung von wenigen, typischerweise etwa 1 bis 2 mm, ggfls. auch etwas mehr. Die Stärke des Drahtes liegt im Submillimeterbereich. Die Wendellänge kann im Bereich weniger Zentimeter, typischerweise 1 bis 3 cm liegen, vorzugsweise bei 1 bis 2 cm. Der Einsatz ist vorzugsweise derart, daß die Wendel 41 nicht vollständig aus dem distalen Ende 14 des Führungsteils 2 herausragt, sondern, wie dies bei der Figur 1 erkennbar ist, teilweise in das Führungsteil 2 hineinreicht, so daß beim Umdrehen der Wendel und der durch diese ausgeübten Förderwirkung Ablagerungsfragmente in das Führungsteil 2 hineingefördert und dort zuverlässig beim Absaugen erfaßt werden können.

Statt der Wendelausbildungen 63 können auch solche in Form einer schraubenförmig, auf der Hülse 62 angeordneten Bürste 67 mit sich radial erstreckenden Borsten 68 vorgesehen sein. Auch hierdurch wird die Förderung der zerkleinerten und im distalen Aufnahmeende 14 der Hülse 2 aufgenommenen Bruchstücke von Ablagerungen zum proximalen Ende der Hülse 2 unterstützt.

Die Arbeitsweise ist vorzugsweise folgende: Zur Entfernung von Ablagerungen in einem Blutgefäß wird dieses zunächst an einer geeigneten Stelle punktiert, anschließend ein Führungsdraht durch die Punktionskanüle in das Blutgefäß eingeführt, die Punktionskanüle über das proximale Ende des Führungsdrahts abgezogen, anschließend ein Katheterbesteck eingeführt und mit diesem oder nach Einführen desselben das als Absaugkatheter dienende Führungsteil 2. Dabei kann sich in diesem, aber zunächst in den Hohlraum des Führungsteils 2 zurückgezogen, schon die Welle 26 mit dem Wendelansatz 41, 61 befinden, oder aber auch nachträglich erst eingeführt werden. Auf jeden Fall wird die Welle 26 mit dem Wendelansatz 41, 61 schließlich in der beschriebenen Art teilweise aus dem distalen Ende 14 des Führungsteils 2 - vorzugsweise um wenige wie 3-10 mm, insbesondere 3-5 mm - herausgeschoben, anschließend die Absaugeinrichtung und der Antriebsmotor 28 in Bewegung gesetzt. Der Motor läuft dabei typischerweise mit Umdrehungszahlen von 20000/ min. (zwanzigtausend/min.) um. Hierdurch wird mittels des Wendelansatzes 41, 61 die Ablagerung im Gefäß abgetragen und durch den Wendelansatz 41, 61 bis zur distalen Öffnung des Führungs- und Absaugteils 2 gefördert, wo es dann durch die Saugeinrichtung wie einer Spritze, Kreiselpumpe od.dgl. ab und damit aus dem Arbeitsbereich des Wendelansatzes 41, 61 fortgesaugt wird. Vor dem Beginn des mechanischen Abtragens und während desselben können die Ablagerungen in der beschriebenen Weise durch Einspritzen von Streptokinase angelöst werden.

Zur Entfernung eines Gallensteins wird die Gallenblase perkutan transhepatisch punktiert. Nach Legen des Führungsdrahts und Entfernen der Punktions-Hohlnadel, wird der Hautbereich aufdilatiert und ein Katheter über den Führungsdraht bis zur Galle geschoben, der, wenn der Führungsdraht in einem Nebenlumen liegt, schon die Welle enthalten kann. Ansonsten wird diese nach Entfernen des Führungsdrahts eingeschoben. Es werden dabei grundsätzlich in der vorstehend beschriebenen Weise Führungs- und Absaugteil 2 und Welle 3 mit Wendelansatz 41, 61 eingeführt. Die Einführung geschieht vorzugsweise unter Durchleuchtung (röntgenologisch) oder Ultraschall-Betrachtung. Im ersten Falle ist vorher ein Kontrastmittel einzuspritzen, was ebenfalls durch das Führungsteil 2 erfolgen kann. Das distale Ende 49 des Wendelansatzes 41, 61 wird auf den Gallenstein gesetzt und der Motor angeschaltet. In diesem Falle wird vorzugsweise ein konischer Wendelansatz 41 (z.B. Figur 3, 7) verwendet. Dieser kann sich aufgrund der hohen Umdrehungsgeschwindigkeit in den Stein bohren (dessen Trägheit und Reibungskräfte ihn an einem Mitdrehen mit der gleichen Geschwindigkeit hindern), wodurch der Stein, insbesondere bei einer konischen Wendelansatzausbildung aufgesprengt werden kann. Die Teile werden weitgehend zerkleinert und die breiartigen Bruchstücke können über die Saugeinrichtung abgesaugt werden, so daß ausgeschlossen ist, daß Fragmente in einzelne Gänge (wie ductus choledochus und ductus pankreaticus) wandern und dort zu Entzündungen führen. Grundsätzlich ist auch ein endoskopisches Vorgehen zur Galle hin möglich.

## Patentansprüche

1. Vorrichtung zum Entfernen von Ablagerungen, wie Plaques in Gefäßen und Steinen in Organen von Lebewesen, mit einem hohlen Führungsteil, einem Motor und einer von diesem angetriebenen, ins Führungsteil ragenden, wobei am Antriebswelle distalen Ende (44) der Antriebswelle (26) ein Wendelansatz (61,63) angeordnet ist, der zumindestens teilweise aus dem distalen Ende (14) des Führungsteils (2) ragt und deren Querschnittabmessungen höchstens dem Innendurchmesser des Führungsteils entspricht, und wobei das distale Ende (29) des Wendelansatzes (41) abgestumpft ausgebildet ist und der äußere Bereich der einzelnen Windungen (47) ebenfalls abgestumpft ist.

2. Vorrichtung nach Anspruch 1, dadurch gekennzeichnet, daß die einzelnen Windungen eines Wendelansatzes (61,63) mit endlichem Abstand zueinander angeordnet sind.

3. Vorrichtung nach Anspruch 1 oder 2, dadurch gekennzeichnet, daß ein Wendelansatz als auf einem axialen Kern (26,62) ausgebildetes Schraubengewinde (63) ausgebildet ist.

4. Vorrichtung nach einem der vorangehenden Ansprüche, dadurch gekennzeichnet, daß das distale (vordere) Ende (14) der Antriebswelle (26) eine blattförmige Verbreiterung (61) aufweist.

5. Vorrichtung nach Anspruch 4, dadurch gekennzeichnet, daß die blattförmige Verbreiterung (61) in einer der Schraubenform (63) entsprechenden Weise um die Symmetrieachse (A) der Antriebswelle (26) in sich gebogen ist.

6. Vorrichtung nach einem der Ansprüche 4 und 5, dadurch gekennzeichnet, daß die Verbreiterung (61) an dem distalen Ende (14) der Antriebswelle (26) einstückig ausgebildet ist, daß der Kern der Schraubgewindeausbildung (63) eine Hülse (62) ist und Antriebswelle (26) und Hülse (62) fest miteinander verbunden sind.

7. Vorrichtung nach einem der Ansprüche 1 bis 3, dadurch gekennzeichnet, daß das distale Ende (14) der Antriebswelle (26) mit einer Kugel versehen ist.

8. Vorrichtung nach einem der Ansprüche 1 bis 7, dadurch gekennzeichnet, daß die Wendelausbildung durch einzelne sich radial erstreckende Borsten gebildet ist.

9. Vorrichtung nach einem der Ansprüche 1 bis 8, dadurch gekennzeichnet, daß das distale Ende (49) des Wendeldrahts in Form einer Acht (51) ausgebildet ist.

10. Vorrichtung nach einem der Ansprüche 1 bis 9, dadurch gekennzeichnet, daß das proximale Ende (7, 21) des Führungsteils (2) gegen die Antriebswelle (26) abgedichtet ist.

11. Vorrichtung nach einem der Ansprüche 1 bis 10, dadurch gekennzeichnet, daß das Führungsteil (2) proximal einen von einem die Antriebswelle (26) aufnehmenden Lumen (3) abzweigenden Abzweig (8) aufweist, der mit einer Saugeinrichtung (18) verbindbar ist.

12. Vorrichtung nach einem der vorangehenden Ansprüche, dadurch gekennzeichnet, daß an dem proximalen Ende (7, 8) des Führungsteils (2) geeignete Befestigungseinrichtungen (23,32) vorgesehen sind.

13. Vorrichtung nach einem der Ansprüche 1 bis 12, dadurch gekennzeichnet, daß das Führungsteil (2) zweilumig ausgebildet ist und neben einem Hauptlumen (3) mit nahezu den gesamten Innenquerschnittsbereich des Führungsteils (2) einnehmenden Querschnitt ein Nebenlumen (4) mit einem wesentlich geringeren Querschnitt aufweist, an dessen proximalem Ende (11) ggfls. über einen Abzweig (9) eine Spritze ansetzbar ist.

14. Vorrichtung nach einem der Ansprüche 1 bis 13, dadurch gekennzeichnet, daß der Wendelansatz (41) durch das Führungsteil (2) bis in den Bereich einer Ablagerung einbringbar ist und durch Rotation des Wendelansatzes (41) die Ablagerung abtragbar ist.

## Claims

1. Apparatus for removing deposits, such as plaque from vessels and stones from organs of living organisms, with a hollow guide part, a motor and a drive shaft projecting into the guide part and driven by the motor, in which on the distal end (44) of the drive shaft (26) is placed a helical attachment (61,63), which at least partly projects out of the distal end (14) of the guide part (2) and whose cross-sectional dimensions at the most correspond to the internal diameter of the guide part and in which the distal end (29) of the helical attachment (41) is truncated and the outer area of the individual turns (47) is also truncated.

2. Apparatus according to claim 1, characterized in that the individual turns of a helical attachment (61,63) are arranged with a finite reciprocal spacing.

3. Apparatus according to claim 1 or 2, characterized in that a helical attachment is constructed as a screw thread (63) formed on an axial core (26,62).

4. Apparatus according to one of the preceding claims, characterized in that the distal (front) end (14) of the drive shaft (26) has a leaf-like widening (61).

5. Apparatus according to claim 4, characterized in that the leaf-like widening (61) is bent around the axis of symmetry (A) of the drive shaft (26) in a manner corresponding to the screw thread (63).

6. Apparatus according to one of the claims 4 and 5, characterized in that the widening (61) at the distal end (14) of the drive shaft (26) is constructed in one piece, that the core of the screw thread (63) is a sleeve (62) and that the drive shaft (26) and the sleeve (62) are firmly interconnected.

7. Apparatus according to one of the claims 1 to 3, characterized in that the distal end (14) of the drive shaft (26) is provided with a ball.

8. Apparatus according to one of the claims 1 to 7, characterized in that the helical construction is formed by individual, radially extending bristles.

9. Apparatus according to one of the claims 1 to 8, characterized in that the distal end (49) of the helical wire is shaped like a figure of eight (51).

10. Apparatus according to one of the claims 1 to 9, characterized in that the proximal end (7,21) of the guide part (2) is sealed against the drive shaft (26).

11. Apparatus according to one of the claims 1 to 10, characterized in that the guide part (2) has at the proximal end a branch (8) branching from the lumen (3) receiving the drive shaft (26) and which can be connected to a suction device (18).

12. Apparatus according to one of the preceding claims, characterized in that suitable fastening devices (23,32) are provided at the proximal end (7,8) of the guide part (2).

13. Apparatus according to one of the claims 1 to 12, characterized in that the guide part (2) has two lumens, namely in addition to a main lumen (3) with a cross-section taking up almost the entire internal cross-sectional area of the guide part (2), a secondary lumen (4) with a much smaller cross-section and to whose proximal end (11) can optionally be fitted by means of a branch (9) a syringe.

14. Apparatus according to one of the claims 1 to 13, characterized in that the helical attachment (41) can be introduced through the guide part (2) into the vicinity of a deposit and the latter can be removed by rotating the helical attachment (41).

## Revendications

1. Dispositif pour enlever des dépôts, tels que plaques dans des vaisseaux et calculs dans des organes d'êtres vivants, avec un élément de guidage creux, un moteur et un arbre moteur, entraîné par ce dernier et pénétrant dans l'élément de guidage, un embout hélicoïdal (61, 63) qui dépasse, en partie du moins, de l'extrémité distale (14) de l'élément de guidage (2), et dont les dimensions en coupe transversale correspondent au maximum au diamètre intérieur de l'élément de guidage, étant disposé sur l'extrémité distale (44) de l'arbre moteur (26), et l'extrémité distale (29) de l'embout hélicoïdal (41) étant émoussée, et la zone externe des différentes spires (47) étant également émoussée.

2. Dispositif suivant la revendication 1, caractérisé en ce que les différentes spires d'un embout hélicoïdal (61, 63) sont disposées avec un écartement mutuel fini.

3. Dispositif suivant l'une des revendications 1 et 2, caractérisé en ce qu'un embout hélicoïdal est réalisé sous forme de pas de vis (63), prévu sur un coeur axial (26, 62).

4. Dispositif suivant l'une quelconque des revendications précédentes, caractérisé en ce que l'extrémité distale (avant) (14) de l'arbre moteur (26) présente un élargissement en forme de feuille (61).

5. Dispositif suivant la revendication 4, caractérisé en ce que l'élargissement en forme de feuille (61) est plié en soi autour de l'axe de symétrie (A) de l'arbre moteur (26), d'une manière correspondante à la forme hélicoïdale (63).

6. Dispositif suivant l'une quelconque des revendications 4 et 5, caractérisé en ce que l'élargissement (61) est réalisé d'une seule pièce sur l'extrémité distale (14) de l'arbre moteur (26), en ce que le coeur de la réalisation en pas de vis (63) est une douille (62), et en ce que l'arbre moteur (26) et la douille (62) sont assemblés fixement entre eux.

7. Dispositif suivant l'une quelconque des revendications 1 à 3, caractérisé en ce que l'extrémité distale (14) de l'arbre moteur (26) est munie d'une bille.

8. Dispositif suivant l'une quelconque des revendications 1 à 7, caractérisé en ce que la configuration hélicoïdale est formée par des crins individuels s'étendant dans le sens radial.

9. Dispositif suivant l'une quelconque des revendications 1 à 8, caractérisé en ce que l'extrémité distale (49) du fil hélicoïdal est réalisée en forme de huit (51).

10. Dispositif suivant l'une quelconque des revendications 1 à 9, caractérisé en ce que l'extrémité proximale (7, 21) de l'élément de guidage (2) est rendue étanche par rapport à l'arbre moteur (26).

11. Dispositif suivant l'une quelconque des revendications 1 à 10, caractérisé en ce que l'élément de guidage (2) présente, dans la zone proximale, une dérivation (8) qui part d'un canal (3), recevant l'arbre moteur (26), et qui peut être raccordée à un dispositif d'aspiration (18).

12. Dispositif suivant l'une quelconque des revendications précédentes, caractérisé en ce que des dispositifs de fixation adéquats (23, 32) sont prévus sur l'extrémité proximale (7, 8) de l'élément de guidage (2).

13. Dispositif suivant l'une quelconque des revendications 1 à 12, caractérisé en ce que l'élément de guidage (2) a une réalisation à deux canaux et présente, en dehors d'un canal principal (3), dont la section occupe la quasi-totalité de la zone de section interne de l'élément (2), un canal secondaire (4) d'une section largement inférieure, sur l'extrémité proximale (11) duquel peut être fixée une seringue, par l'intermédiaire d'une dérivation (9) le cas échéant.

14. Dispositif suivant l'une quelconque des revendications 1 à 13, caractérisé en ce que l'embout hélicoïdal (41) peut être introduit par l'élément de guidage (2) jusque dans la zone d'un dépôt, enlevé par rotation de l'embout hélicoïdal (41).
